# EUROPÄISCHE PATENTSCHRIFT

(11) **EP 0 753 088 B1**
(45) Veröffentlichungstag und Bekanntmachung des Hinweises auf die Patenterteilung: **12.11.1997**
(21) Anmeldenummer: 95913884.3
(22) Anmeldetag: 17.03.1995
(51) Int. Cl.: D02J 1/08, D02G 1/16, G01N 33/36

(54) **VERFAHREN UND VORRICHTUNG ZUM PRÜFEN VON VERWIRBELTEN FILAMENTGARNEN**
METHOD AND DEVICE FOR CHECKING SWIRLED-FILAMENT YARNS
PROCEDE ET DISPOSITIF POUR LE CONTROLE DE FILES A FILAMENTS TORSADES

(30) Priorität: 26.03.1994 DE 4410571
(43) Veröffentlichungstag der Anmeldung: 15.01.1997
(73) Patentinhaber: TEMCO TEXTILMASCHINENKOMPONENTEN GmbH & Co. KG, 97762 Hammelburg (DE); DEUTSCHE INSTITUTE FÜR TEXTIL- UND FASERFORSCHUNG STUTTGART Stiftung des öffentlichen Rechts, 73770 Denkendorf (DE)
(72) Erfinder: RIPPSTEIN, Klaus, D-97762 Hammelburg (DE); WEINSDÖRFER, Helmut, D-72124 Pliezhausen (DE)
(74) Vertreter: Matschkur, Götz, Lindner Patent- und Rechtsanwälte
(86) Internationale Anmeldenummer: EP9500996
(87) Internationale Veröffentlichungsnummer: WO9526434

(56) Entgegenhaltungen:
- EP-A- 0 465 842
- EP-A- 0 572 756
- US-A- 4 990 793

## Beschreibung

Die Erfindung betrifft ein Verfahren zum Prüfen von verwirbelten Filamentgarnen hinsichtlich ihrer Verwirbelungsintensität beziehungsweise auf unzulässig lange, unverwirbelte oder nicht hinreichend verwirbelte Garnabschnitte während des Verwirbelns der Garne mittels einer Verwirbelungseinrichtung.

Das Verwirbeln hat den Zweck, den Zusammenhalt der einzelnen Filamente von Multifilamentgarnen und damit deren weitere Verarbeitbarkeit zu verbessern. Verwirbelungsvorrichtungen sind bekannt (zum Beispiel deutsche Patentschrift 37 11 759). Diese Verwirbelungseinrichtungen arbeiten mittels eines Luftstrahls, der die zunächst parallelliegenden einzelnen Filamente des Garns mechanisch miteinander verbindet, wodurch Verwirbelungsknoten mit dazwischenliegenden offenen, unverwirbelten Garnabschnitten entstehen. Den Mechanismus der Verwirbelung kann man sich so vorstellen, daß der scharfe, aus einer Blasdüse austretende Luftstrom das Filamentgarn zerteilt, das heißt er öffnet den Faden und "verbläst" die einzelnen Filamente, wobei sich Teilstromwirbel ausbilden, welche die Filamente lokal miteinander verdrehen und dabei die entsprechenden Verwirbelungsknoten bilden. Bei einwandfreier Verwirbelung weisen die Verwirbelungsknoten etwa gleiche Abstände voneinander auf, das heißt, sie wiederholen sich nahezu periodisch. Treten Störungen bei der Verarbeitung auf, so kommt es zu unzulässig langen, unverwirbelten oder nicht hinreichend verwirbelten Garnabschnitten.

Es ist bekannt, verwirbelte Filamentgarne im Labor einer Prüfung hinsichtlich ihrer Verwirbelungsstellen zu unterziehen. In den letzten Jahren hat sich die mechanische Abtastung durchgesetzt. Dabei wird das zu untersuchende Filamentgarn langsam durch einen Meßspalt gezogen, der die Dicke des Fadens abtastet. Parallelliegende Filamente werden in Bändchenform flachgedrückt. Die kompakten, verwirbelten Stellen (Verwirbelungsknoten) lassen dies nicht zu; sie werden als Dickstellen registriert. Durch eine kontinuierliche und lückenlose Abtastung ist es daher möglich, eine genaue Analyse der Verwirbelungscharakteristik durchzuführen. Allerdings läßt sich dieses bekannte Verfahren nur an exemplarischen Prüffäden durchführen, da die Prüfgeschwindigkeit relativ niedrig ist und daher während der laufenden Produktion der verwirbelten Filamentgarne nicht angewendet werden kann.

Der Erfindung liegt daher die Aufgabe zugrunde, ein Verfahren zur Qualitätskontrolle der Verwirbelungsintensität von verwirbelten Filamentgarnen, auch hinsichtlich unzulässig langer, unverwirbelter oder nicht hinreichend verwirbelter Garnabschnitte, anzugeben, das während des Verwirbelns der Garne durchführbar ist, so daß während der Herstellung auch bei hohen Produktionsgeschwindigkeiten eine kontinuierliche Qualitätskontrolle durchgeführt werden kann.

Diese Aufgabe wird erfindungsgemäß dadurch gelöst, daß im Bereich der Verwirbelungseinrichtung eine Schwingungsmessung des Garnes erfolgt, deren Ergebnis für die Prüfung ausgewertet wird. Erfindungsgemäß ist also vorgesehen, daß Garnschwingungen im Bereich der Verwirbelungseinrichtung erfaßt werden, wobei überraschenderweise gefunden wurde, daß das Ergebnis dieser Schwingungsmessung eine Aussage darüber zuläßt, ob eine einwandfreie, kontinuierliche Verwirbelung vorliegt, oder ob Fehlstellen vorliegen, das heißt, Garnabschnitte, die nicht oder nicht hinreichend verwirbelt wurden.

Nach einer bevorzugten Ausführungsform der Erfindung erfolgt die Schwingungsmessung mittels eines mechanische Schwingungen erfassenden Schwingungsaufnehmers. Dieser registriert die im mit hoher Geschwindigkeit bewegten Filamentgarn auftretenden Schwingungen, seien es Transversal-Schwingungen und/oder Longitudinal-Schwingungen.

Alternativ ist es auch möglich, daß die beim Verwirbeln auftretenden Schwingungen des Garnes mittels einer optischen Schwingungsmeßeinrichtung erfaßt werden. Das Erfassen dieser Schwingungen kann daher berührungslos erfolgen.

Ferner ist es durch eine akustische Schwingungsmeßeinrichtung ebenfalls möglich, berührungslos die vom Garn erzeugten Luftschwingungen zu erfassen.

Schließlich ist es nach einem weiteren Ausführungsbeispiel möglich, die Schwingungen des Garnes mittels einer Fadenzugkraft-Meßeinrichtung zu erfassen. Das Niveau der Fadenzugkraft an sich enthält bereits eine Information über die Verwirbelungsintensität. Der eigentlichen Fadenzugkraft ist aber aufgrund der sich einstellenden Schwingungen eine Schwingungskomponente überlagert, die zu einer entsprechenden Fadenzugkraftänderung (Longitudinal-Schwingung) führt, welche für die Prüfung auf einwandfreie Verwirbelung als besonders geeignet herangezogen werden kann.

Für die Auswertung der erfindungsgemäßen Schwingungsmessung ist es erforderlich, die Schwingungsfrequenz und/oder die Schwingungsamplitude heranzuziehen. Die Frequenz der Schwingungen liegt -je nach Garnart und Produktionsgeschwindigkeit- im Kilohertzbereich, beispielsweise bei etwa einem Kilohertz. Ist diese Schwingungszahl kontinuierlich über die Länge des Filamentgarnes vorhanden, so liegt eine einwandfreie Verwirbelung vor. Fehlstellen im Garn, das heißt, unzulässig große Knotenabstände, wirken sich auf die Frequenz und/oder die Amplitude aus und können daher mittels geeigneter technischer Einrichtungen sehr leicht identifiziert werden.

Auf diese Art und Weise kann automatisch während der Herstellung des verwirbelten Multifilamentgarnes die Qualitätskontrolle durchgeführt werden.

Die Erfindung betrifft ferner eine Vorrichtung zur Prüfung von verwirbelten Filamentgarnen hinsichtlich ihrer Verwirbelungsintensität bzw. auf unzulässig lange, unverwirbelte oder nicht hinreichend verwirbelte Garnabschnitte während des Verwirbelns der Garne mittels einer Verwirbelungseinrichtung, wobei im Bereich der Verwirbelungseinrichtung eine Schwingungserfassungs-Einrichtung angeordnet ist, die Garnschwingungen zur Durchführung der Prüfung erfaßt.

Die Schwingungserfassungs-Einrichtung kann an/in der Verwirbelungseinrichtung angeordnet sein.

Es ist möglich, die Schwingungserfassungs-Einrichtung nicht direkt an der Verwirbelungseinrichtung anzuordnen, sondern stromaufwärts oder aber stromabwärts der Verwirbelungseinrichtung zu installieren. So kann die Schwingungserfassungs-Einrichtung beispielsweise an vorzugsweise beidseitig der Verwirbelungseinrichtung angeordneten Garnführern angeordnet sein. "Stromaufwärts" der Verwirbelungseinrichtung bedeutet, daß sich die Schwingungserfassungs-Einrichtung im Einlaufbereich (mit Bezug auf die Bewegungsrichtung des Garnes) der Verwirbelungseinrichtung befindet; "stromabwärts" hat die Bedeutung, daß die Schwingungserfassungs-Einrichtung hinsichtlich der Garnbewegung der Verwirbelungseinrichtung nachgeschaltet ist.

Die Schwingungserfassungs-Einrichtung weist vorzugsweise einen Kraftaufnahme-Sensor auf, der -durch die Schwingungen bewirkte- mechanische Kräfte erfaßt und in ein elektrisches Signal für die Auswertung umwandelt. Der Kraftaufnahme-Sensor kann bevorzugt als Piezo-Sensor ausgebildet sein. Ein derartiger Sensor erfaßt die dynamischen Änderungen, das heißt, er erfaßt nicht ein möglicherweise vorhandenes Grundniveau einer Kraft, sondern nur die zeitlichen Änderungen. Die einer zeitlichen Änderung unterliegenden Schwingungen lassen sich daher erfassen. Ein solcher Sensor ist auch geeignet, das Vorhandensein des Fadens festzustellen, das heißt den Fadenlauf zu überwachen.

Alternativ ist es jedoch auch möglich, als Kraftaufnahme-Sensor Dehnmeßstreifen einzusetzen. Diese Dehnmeßstreifen sind in der Lage, nicht nur dynamische Änderungen, sondern auch das Grundniveau zu erfassen, so daß beispielsweise neben der Schwingungserfassung auch eine Fadenzugkraft-Erfassung erfolgen kann.

Insbesondere ist es möglich, daß als Kraftaufnahme-Sensor ein Fadenzugkraftmesser eingesetzt wird. Dieser ist derart auszubilden, und sein Meßergebnis derart auszuwerten, daß nicht nur der Mittelwert und sein Verlauf, sondern vor allem auch die dem Mittelwert der Fadenzugkraft überlagerten Zugkraftschwingungen, die durch die Fadenschwingungen bewirkt sind, der Prüfung auf unzulässig lange, unverwirbelte oder nicht hinreichend verwirbelte Garnabschnitte zugrundegelegt werden.

Vorteilhafte Weiterbildungen der Erfindung sind in den weiteren Unteransprüchen gekennzeichnet.

Die Zeichnungen veranschaulichen die Erfindung anhand von Ausführungsbeispielen und zwar zeigt:
- Figur 1: eine Verwirbelungseinrichtung, die eine Schwingungserfassungs-Einrichtung aufweist,
- Figur 2: ein weiteres Ausführungsbeispiel der Erfindung,
- Figur 3: ein weiteres Ausführungsbeispiel der Erfindung und
- Figur 4: eine Detailansicht aus Figur 1.

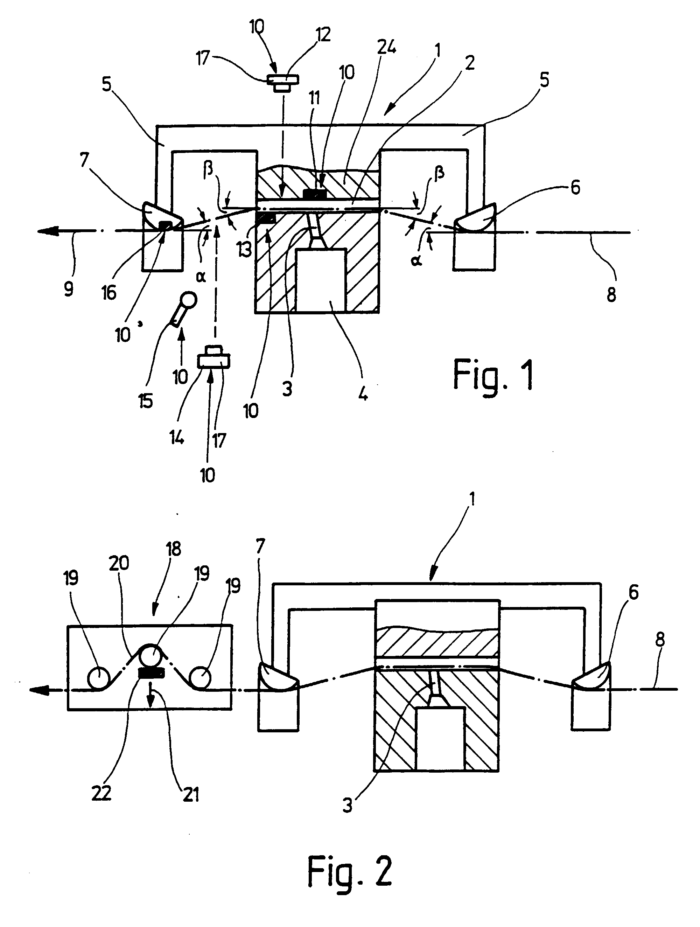

Die Figur 1 zeigt eine Verwirbelungseinrichtung 1, die einen Garnkanal 2 aufweist, in den eine Blasdüse 3 mündet, die an einen Druckluftkanal 4 angeschlossen ist.

Integral mit der Verwirbelungseinrichtung 1 sind - beidseitig des Garnkanals 2- an Haltearmen 5 Garnführer 6,7 ausgebildet. Ein Filamentgarn 8 liegt - in Garntransportrichtung gesehen (Pfeil 9)- an dem stromaufseitig zum Garnkanal 2 gelegenen Garnführer 6 an und wird von dort aus um einen Winkel α umgelenkt und tritt dann unter nochmaliger Umlenkung in entgegengesetzter Richtung um den Winkel β in den Garnkanal 2 ein. Das Filamentgarn 8 durchläuft den Garnkanal 2 und tritt an dessen gegenüberliegendem Ende unter Umlenkung des Winkels β aus und wird dem Garnführer 7 zugeführt, an dem wiederum -in entgegengesetzter Richtung zum Winkel β- eine Umlenkung um den Winkel α erfolgt.

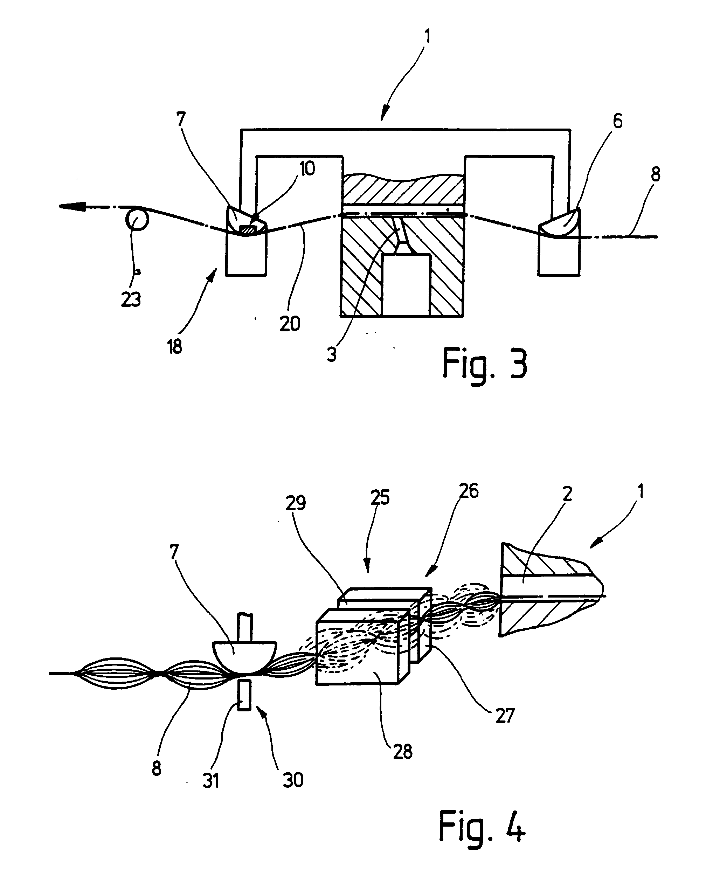

Während des Betriebs werden die zunächst etwa parallel zueinander laufenden Filamente des Filamentgarns 8 mit hoher Geschwindigkeit in Richtung des Pfeiles 9 bewegt, wobei sie einem scharfen Blasstrahl der Blasdüse 3 ausgesetzt werden und hierdurch verwirbeln, wodurch sich Verwirbelungsknoten bilden. Betrachtet man beispielsweise den zwischen dem Ende des Garnkanals 2 und dem Garnführer 7 liegenden Abschnitt des Filamentgarns 8, der in der Figur 4 detailliert dargestellt ist, so wird deutlich, daß das Filamentgarn 8 in diesem Bereich Garnschwingungen ausführt. Diese sind mit gestrichelter Linie in Figur 4 angedeutet. Diese Schwingungen werden zur Prüfung des verwirbelten Filamentgarns 8 auf unzulässig lange, unverwirbelte oder nicht hinreichend verwirbelte Garnabschnitte während des Verwirbelns des Filamentgarns 8 von einer Schwingungserfassungs-Einrichtung 10 erfaßt. In der Figur 1 sind sechs verschiedene Ausführungsbeispiele der Schwingungserfassungs-Einrichtung 10 dargestellt, wobei in der Praxis jedoch stets nur eine dieser Einrichtungen eingesetzt werden wird. Mit dem Bezugszeichen 11 ist eine Schwingungserfassungs-Einrichtung 10 gekennzeichnet, die hier im Bereich eines Düsendeckels 24 des Garnkanals 2 liegt. Mit dem Bezugszeichen 12 ist eine Schwingungserfassungs-Einrichtung 10 gekennzeichnet, die ebenfalls dem Garnkanal 2 zugeordnet ist und auf optischem Wege arbeitet. Das Bezugszeichen 13 kennzeichnet eine Schwingungserfassungs-Einrichtung 10, die an der stromabseitigen Kante des Garnkanals 2, also im Bereich des Garnkanalausgangs, liegt. Mit 14 ist eine Schwingungserfassungs-Einrichtung gekennzeichnet, die auf optischem Wege den Abschnitt des Filamentgarns 8 abtastet, der zwischen dem Ausgang des Garnkanals 2 und dem Garnführer 7 liegt. Das Bezugszeichen 15 kennzeichnet eine Schwingungserfassungs-Einrichtung 10, die auf akustischem Wege die Schwingungen erfaßt. Schließlich ist mit dem Bezugszeichen 16 eine Schwingungserfassungs-Einrichtung 10 gekennzeichnet, die am Garnführer 7 angeordnet ist.

An dieser Stelle sei erwähnt, daß in der Figur 1 lediglich Schwingungserfassungs-Einrichtungen 10 angegeben sind, die entweder dem Garnkanal 2 zugeordnet oder stromabwärts des Garnkanals 2 angeordnet sind. Es ist -nach weiteren Ausführungsbeispielen der Erfindung- auch möglich, daß sich die Schwingungserfassungs-Einrichtungen 10 mit den Bezugszeichen 13 bis 16 im stromaufwärtigen Bereich befinden, also an der eingangsseitigen Kante des Garnkanals 2, im Bereich des Abschnitts des Filamentgarns 8 zwischen dem Garnführer 6 und dem Garnkanal 2 sowie am Garnführer 6 selbst.

Zusätzlich ist es auch möglich, daß eine oder mehrere Schwingungserfassungs-Einrichtungen 10 an separaten Elementen angeordnet sind, die im Bereich der Verwirbelungseinrichtung 1 liegen und vom Filamentgarn 8 beaufschlagt werden, so daß sie Schwingungen des Filamentgarns 8 aufnehmen können.

Grundsätzlich sind -nach den gezeigten Ausführungsbeispielen- vier verschiedene Arten von Schwingungserfassungs-Einrichtungen 10 angegeben. Sie können einen Kraftaufnahme-Sensor zur Schwingungsmessung aufweisen, der bevorzugt als Piezo-Sensor oder als Dehnmeßstreifen-Sensor ausgebildet ist. Im Falle des Piezo-Sensors werden nur dynamische Vorgänge erfaßt; der Dehnmeßstreifen erfaßt sowohl die dynamischen als auch die absoluten Werte.

Die Schwingungserfassungs-Einrichtung 11 weist beispielsweise einen Piezo-Sensor auf, der die Schwingungen des Filamentgarns 8 im Garnkanal 2 registriert. Die Schwingungserfassungs-Einrichtung 12 arbeitet auf optischem Wege, das heißt, sie ist als optische Schwingungsmeßeinrichtung 17 ausgebildet, die das Innere des Garnkanals 2 erfaßt und auf diese Art und Weise die Schwingungen registriert. Die Schwingungserfassungs-Einrichtung 13 kann wiederum als Piezo-Sensor ausgebildet sein, das heißt, es werden die an der Kante des Garnkanals 2 auftretenden, durch die Schwingungen des Filamentgarns 8 hervorgerufenen Kräfte aufgenommen.

Die Schwingungserfassungs-Einrichtung 14 ist wiederum als optische Schwingungsmeßeinrichtung 17 ausgebildet, die den zwischen dem Ausgang des Garnkanals 2 und dem Garnführer 7 liegenden Abschnitt des Filamentgarns 8 erfaßt und auf diese Art und Weise die Schwingungen registriert. Die Schwingungserfassungs-Einrichtung 15 arbeitet auf akustischem Wege. Sie weist einen Schwingungsaufnehmer, insbesondere ein Mikrofon auf, das die Schwingungen des Filamentgarnes 8 im Luftstrom akustisch registriert. Schließlich weist die Schwingungserfassungs-Einrichtung 16 wiederum einen mechanischen Kraftaufnehmer auf, der insbesondere als Piezo-Sensor ausgebildet sein kann.

In der Figur 2 ist ein weiteres Ausführungsbeispiel erläutert, das sich -gegenüber dem der Figur 1- dadurch unterscheidet, daß der Verwirbelungseinrichtung 1 ein Fadenzugkraftmesser 18 nachgeschaltet ist. Nach einem anderen, nicht dargestellten Ausführungsbeispiel ist es jedoch auch möglich, daß der Fadenzugkraftmesser 18 der Verwirbelungseinrichtung 1 vorgeschaltet ist. Das Filamentgarn 8 wird mittels dreier Umlenkungen 19 des Fadenzugkraftmessers 18 entlang eines Bogens 20 umgelenkt, so daß -aufgrund der Fadenzugkraft- auf die mittlere Umlenkung 19 eine Kraft in Richtung des Pfeiles 21 -in Abhängigkeit von der Fadenzugkraft (umgangssprachlich oft auch als Fadenspannung bezeichnet)- ausgeübt wird. Dieser mittleren Umlenkung 19 ist ein Kraftaufnehmer 22 zugeordnet, der vorzugsweise als Dehnmeßstreifen ausgebildet ist. Er registriert einerseits die Fadenzugkraft und andererseits die dieser Fadenzugkraft überlagerten Kraftschwankungen, die durch die Schwingungen des Filamentgarns 8 ausgelöst werden. Auf diese Art und Weise ist es möglich, die Schwingungen zu erfassen und zur Prüfung des Filamentgarns 8 auf unzulässig lange, unverwirbelte oder nicht hinreichend verwirbelte Garnabschnitte heranzuziehen. Der Kraftaufnehmer muß so ausgebildet sein, daß er Schwingungen im Kilohertzbereich (bis etwa 5 kHz) messen kann.

Die Figur 3 zeigt ein weiteres Ausführungsbeispiel, bei dem das Filamentgarn 8 entsprechend der Anordnung der Figur 1 geführt ist, wobei -nachgeschaltet zum Garnführer 7- jedoch eine Umlenkung 23 vorgesehen ist, die -ebenso wie beim Ausführungsbeispiel der Figur 2- einen Bogen 20 -bedingt durch den Garnführer 7- des Filamentgarns 8 zwischen dem Ausgang des Garnkanals 2 und der Umlenkung 23 erzeugt. Eine Schwingungserfassungs-Einrichtung 10, die vorzugsweise als Dehnmeßstreifen ausgebildet ist, befindet sich am Garnführer 7, wodurch eine Anordnung geschaffen wird, die der der Figur 2 entspricht, also einen Fadenzugkraftmesser 18 bildet, der jedoch nicht als separates Bauteil vorliegt, sondern in die Verwirbelungseinrichtung 1 quasi integriert ist und -zwecks Konstanthaltung des Bogens 20 beziehungsweise des Umschlingungswinkels am Garnführer 7- zusätzlich lediglich die erwähnte Umlenkung 23 benötigt.

Die Figur 4 zeigt zwei weitere Möglichkeiten, die Schwingungen des Garns 8 zu messen. Es ist eine Schwingungserfassungs-Einrichtung 25 vorgesehen, die als kapazitiver Sensor 26 ausgebildet ist. Der kapazitive Sensor 26 weist zwei Kondensatorplatten 27 und 28 auf, die zwischen sich ein Dielektrikum 29 ausbilden. Durch die Schwingungen des Filamentgarnes 8, das zwischen den Kondensatorplatten 27 und 28 verläuft, ändert sich die dem kapazitiven Sensor 26 zuzurechnende Fadenlänge, wodurch sich eine Änderung des Dielektrikums einstellt. Die Fadenschwingungen führen also zu entsprechenden Änderungen des Dielektrikums, die erfaßt werden können und daher eine Aussage über die Schwingungen des Filamentgarnes 8 und insofern über dessen Eigenschaften zulassen.

In der Figur 4 ist ferner eine weitere Schwingungserfassungs-Einrichtung 30 angegeben, die als optischer Sensor 31 ausgebildet ist. Vorzugsweise ist der optische Sensor 31 im Bereich des Garnführers 7 angeordnet, kann jedoch auch noch weiter stromab angeordnet sein. Entscheidend ist, daß sich die Schwingungserfassungs-Einrichtung 30 dort befindet, wo bereits eine Verwirbelung des Filamentgarns 8 stattgefunden hat. Der optische Sensor 31 kann beispielsweise als Fotodiodenzeile, CCD-Zeile, als Lichtschranke oder Laser-Einheit ausgebildet sein. Aufgrund dessen, daß die Verwirbelungsknoten Dikkenänderungen des Filamentgarns 8 bewirken und das Filamentgarn 8 einseitig am Fadenführer 7 abgestützt ist, wird sich im Zuge des Fadentransports der Abstand, der dem optischen Sensor 31 zugewandten Seite des Filamentgarns 8, zum optischen Sensor 31 entsprechend der Fadendicke verändern, so daß - aufgrund des Fadentransports- dieser optische Sensor 31 Schwingungen registriert, die wiederum eine Aussage für die Qualität, insbesondere die Verwirbelungsintensität gestatten. Der optische Sensor 31 wirkt somit quasi als Abstandsmesser und läßt einen Rückschluß auf die Fadendicke zu.

## Patentansprüche

1. Verfahren zum Prüfen von verwirbelten Garnen, Garnmischungen, Foliengarnen, bevorzugt Filamentgarnen (8) hinsichtlich ihrer Verwirbelungsintensität beziehungsweise auf unzulässig lange, unverwirbelte oder nicht hinreichend verwirbelte Garnabschnitte während des Verwirbelns der Garne (8) mittels einer Verwirbelungseinrichtung (1), dadurch gekennzeichnet, daß in der Verwirbelungseinrichtung (1) oder in deren Bereich eine Schwingungsmessung des Garns (8) erfolgt, deren Ergebnis für das Prüfen ausgewertet wird.

2. Verfahren nach Anspruch 1, dadurch gekennzeichnet, daß die Schwingungsmessung mittels eines mechanische Schwingungen erfassenden Schwingungsaufnehmers durchgeführt wird.

3. Verfahren nach Anspruch 1, dadurch gekennzeichnet, daß die Schwingungen des Garns (8) mittels einer optischen Schwingungsmeßeinrichtung (17) erfaßt werden.

4. Verfahren nach Anspruch 1, dadurch gekennzeichnet, daß die Schwingungen des Garns (8) mittels einer akustischen Schwingungsmeßeinrichtung (15) erfaßt werden.

5. Verfahren nach Anspruch 1, dadurch gekennzeichnet, daß die Schwingungen des Garns (8) mittels einer Fadenzugkraft-Meßeinrichtung (18) erfaßt werden.

6. Verfahren nach Anspruch 1, dadurch gekennzeichnet, daß die Schwingungsmessung mittels eines optischen Sensors (31) durchgeführt wird, der seinen sich entsprechend der Dicke des Filamentgarns (8) ändernden Abstand zum Filamentgarn (8) als Schwingungen registriert.

7. Vorrichtung zur Prüfung von verwirbelten Filamentgarnen (8) auf unzulässig lange, unverwirbelte oder nicht hinreichend verwirbelte Garnabschnitte während des Verwirbelns der Garne mittels einer Verwirbelungseinrichtung (1), dadurch gekennzeichnet, daß in der Verwirbelungseinrichtung (1) oder in deren Bereich eine Schwingungserfassungs-Einrichtung (10 bis 18 und 25, 30) angeordnet ist, die zur Durchführung der Prüfung Garnschwingungen oder als Schwingung registrierbare Änderungen ihres Abstands zum Garn erfaßt.

8. Vorrichtung nach Anspruch 7, dadurch gekennzeichnet, daß die Schwingungserfassungs-Einrichtung (10,11,12,13) in beziehungsweise an der Verwirbelungseinrichtung (1) angeordnet ist.

9. Vorrichtung nach Anspruch 7, dadurch gekennzeichnet, daß die Schwingungserfassungs-Einrichtung (10,14,15,16, Kraftaufnehmer 22) zum Fadenlauf stromaufwärts der Verwirbelungseinrichtung (1) angeordnet ist.

10. Vorrichtung nach Anspruch 7, dadurch gekennzeichnet, daß die Schwingungserfassungs-Einrichtung (10,14,15,16, Kraftaufnehmer 22,25,30) zum Fadenlauf stromabwärts der Verwirbelungseinrichtung (1) angeordnet ist.

11. Vorrichtung nach einem der vorhergehenden Ansprüche, dadurch gekennzeichnet, daß die Schwingungserfassungs-Einrichtung (10,11,13,16, Kraftaufnehmer 22) einen Kraftaufnahme-Sensor zur Schwingungsmessung aufweist.

12. Vorrichtung nach Anspruch 11, dadurch gekennzeichnet, daß der Kraftaufnahme-Sensor als Piezo-Sensor ausgebildet ist.

13. Vorrichtung nach Anspruch 11, dadurch gekennzeichnet, daß der Kraftaufnahme-Sensor als Dehnmeßstreifen-Sensor, Induktiv-Sensor, Hall-Sensor oder kapazitiver Sensor ausgebildet ist.

14. Vorrichtung nach Anspruch 11, dadurch gekennzeichnet, daß der Kraftaufnahme-Sensor als Fadenzugkraftmesser (18) ausgebildet ist.

15. Vorrichtung nach einem der Ansprüche 11-14, dadurch gekennzeichnet, daß die Verwirbelungseinrichtung (1) einen Garnkanal (2,24) aufweist, und da'der Kraftaufnahme-Sensor im Bereich des Garnkanals (2,24) ausgebildet ist.

16. Vorrichtung nach einem der Ansprüche 11-14, dadurch gekennzeichnet, daß die Verwirbelungseinrichtung (1) zumindest einen Garnführer (6,7) aufweist, und daß der Kraftaufnahme-Sensor an mindestens einem Garnführer (6,7) ausgebildet ist.

17. Vorrichtung nach einem der Ansprüche 7-10, dadurch gekennzeichnet, daß die Schwingungserfassungs-Einrichtung (25,30) ein kapazitiver (26) oder ein optischer (31) Sensor ist.

18. Vorrichtung nach einem der vorangehenden Ansprüche 7, 8 oder 10 bis 17, dadurch gekennzeichnet, daß die Schwingungserfassungs-Einrichtung (18;30,31) nach einem Garnführer (7) angeordnet ist, der zum Fadenlauf stromabwärts der Verwirbelungseinrichtung (1) angeordnet ist.

## Claims

1. Method of testing tangled yarns, yarn mixtures, film yarnsl and preferably filament yarns (8) in respect of their tangling intensity or for unacceptably long, non-tangled or insufficiently tangled yarn sections during the tangling of the yarns (8) by means of a tangling device (1), characterised in that in the tangling device (1) or in the regions thereof the vibration of the yarn (8) is measured and the result is evaluated for testing.

2. Method according to claim 1, characterised in that the vibration measurement is carried out by means of a vibration sensor which detects mechanical vibrations.

3. Method according to claim 1, characterised in that the vibrations of the yarn (8) are detected by means of an optical vibration measuring device (17).

4. Method according to claim 1, characterised in that the vibrations of the yarn (8) are detected by means of an acoustic-vibration measuring device (15).

5. Method according to claim 1, characterised in that the vibrations of the yarn (8) are detected by means of a thread tension-measuring device (18).

6. Method according to claim 1, characterised in that the vibration measurement is carried out by means of an optical sensor, which measures as vibrations its variable distance from the filament yarn (8) according to the thickness of the filament yarn (8).

7. Apparatus for testing tangled filament yarns (8) for unacceptably long, non-tangled or insufficiently tangled yarn sections during tangling of the yarns by means of tangling device (1), characterised in that in the tangling devide (1) or in the region thereof, a vibration detection device (10 to 18 and 25, 30) is provided, which in order to carry out the test detects changes in its distance from the yarn which are registrable as a vibration.

8. Apparatus according to claim 7, characterised in that the vibration detection device (10, 11, 12, 13) is disposed in or on the tangling device (1).

9. Apparatus according to claim 7, characterised in that the vibration detection device (10, 14, 15, 16, power sensor 22) is disposed upstream of the tangling device (1) ) in the direction of travel of the thread.

10. Apparatus according to claim 7, characterised in that the vibration detection device (10, 14, 15, 16, power sensor 22, 25, 30) is disposed downstream of the tangling device (1) ) in the direction of travel of the thread.

11. Apparatus according to one of the preceding claims, characterised in that the vibration detection device (10, 11, 13, 16, power sensor 22) has a power take-up sensor for measuring the vibration.

12. Apparatus according to claim 11, characterised in that the power take-up sensor is in the form of a piezoelectric sensor.

13. Apparatus according to claim 11, characterised in that the power take-up sensor is in the form of an expanding measuring strip sensor, an inductive sensor, Hall sensor or capacitive sensor.

14. Apparatus according to claim 11, characterised in that the power take-up sensor is in the form of a thread tension sensor (18).

15. Apparatus according to one of claims 11-14, characterised in that the tangling device (1) ) has one yarn channel (2, 24) and the power take-up sensor is formed in the region of the yarn channel (2, 24).

16. Apparatus according to one of claims 11-14, characterised in that the tangling device (1) ) has at least one yarn guide (6, 7) and the power take-up sensor is formed on at least one yarn guide (6, 7).

17. Apparatus according to one of claims 7-10, characterised in that the vibration detection device (25, 30) is a capacitive (26) or an optical (31) sensor.

18. Apparatus according to one of the preceding claims 7, 8 or 10 to 17, characterised in that the vibration detection device (18; 30, 31) is mounted after a yarn guide (7) which is disposed downstream of the tangling device (1) ) in the direction of travel of the thread.

## Revendications

1. Procédé pour contrôler des fils, des mélanges de fils, des fils fibrillés, de préférence des fils de filaments (8), torsadés, en ce qui concerne leur intensité de torsion ou sur la présence de tronçons de fils non torsadés ou insuffisamment torsadés, de longueurs inadmissibles, pendant la torsion des fils (8) à l'aide d'un dispositif de torsion (1), caractérisé en ce que, dans le dispositif de torsion (1) ou dans son voisinage, est effectué une mesure d'oscillations du fil (8), dont le résultat est exploité pour le contrôle.

2. Procédé selon la revendication 1, caractérisé en ce que la mesure d'oscillations est exécutée à l'aide d'un enregistreur d'oscillations qui détecte des oscillations mécaniques.

3. Procédé selon la revendication 1, caractérisé en ce que les oscillations du fil (8) sont détectées à l'aide d'un dispositif optique de mesure d'oscillations (17).

4. Procédé selon la revendication 1; caractérisé en ce que les oscillations du fil (8) sont détectées à l'aide d'un dispositif acoustique de mesure d'oscillations (15).

5. Procédé selon la revendication 1, caractérisé en ce que les oscillations du fil (8) sont détectées à l'aide d'un dispositif (18) de mesure de la force de traction du fil.

6. Procédé selon la revendication 1, caractérisé en ce que la mesure d'oscillations est exécutée à l'aide d'un capteur optique (31), qui enregistre, sous forme d'oscillations, sa distance par rapport au fil de filaments (8), qui varie en fonction de l'épaisseur du fil de filaments (8).

7. Dispositif pour contrôler des fils de filaments torsadés (8) sur la présence de tronçons non torsadés ou insuffisamment torsadés, de longueurs inadmissibles, pendant la torsion des fils à l'aide d'un dispositif de torsion (1), caractérisé en ce que, dans le dispositif de torsion (1) ou dans son voisinage, est disposé un dispositif de détection d'oscillations (10 à 18 et 25, 30) qui, pour l'exécution du contrôle, détecte des oscillations du fil ou des variations de sa distance par rapport au fil, qui peuvent être enregistrées en tant qu'oscillations.

8. Dispositif selon la revendication 7, caractérisé en ce que le dispositif de détection d'oscillations (10, 11, 12, 13) est disposé dans ou sur le dispositif de torsion (1).

9. Dispositif selon la revendication 7, caractérisé en ce que le dispositif de détection d'oscillations (10, 14, 15, 16, transducteur de force 22) est disposé en amont du dispositif de torsion (1) par rapport à la direction de circulation du fil.

10. Dispositif selon la revendication 7, caractérisé en ce que le dispositif de détection d'oscillations (10, 14, 15, 16, transducteur de force 22, 25, 30) est disposé en aval du dispositif de torsion (1) par rapport à la direction de circulation du fil.

11. Dispositif selon l'une des revendications précédentes, caractérisé en ce que le dispositif de détection d'oscillations (10, 11, 13, 16, transducteur de force 22) comporte un capteur d'enregistrement de force pour la mesure d'oscillations.

12. Dispositif selon la revendication 11, caractérisé en ce que le capteur d'enregistrement de force est réalisé sous la forme d'un capteur piézoélectrique.

13. Dispositif selon la revendication 11, caractérisé en ce que le capteur d'enregistrement de force est réalisé sous la forme d'un capteur à jauge extensométrique, d'un capteur inductif, d'un capteur de Hall ou d'un capteur capacitif.

14. Dispositif selon la revendication 11, caractérisé en ce que le capteur d'enregistrement de force est réalisé sous la forme d'un appareil (18) de mesure de la force de traction du fil.

15. Dispositif selon l'une des revendications 11-14, caractérisé en ce que le dispositif de torsion (1) comporte un canal (2, 24) de passage du fil et en ce que le capteur d'enregistrement de force est formé dans la zone du canal (2, 24) de passage du fil.

16. Dispositif selon l'une des revendications 11-14, caractérisé en ce que le dispositif de torsion (1) comporte au moins un guide-fil (6, 7) et en ce que le capteur d'enregistrement de force est formé sur au moins un guide-fil (6, 7).

17. Dispositif selon l'une des revendications 7-10, caractérisé en ce que le dispositif de détection d'oscillations (25, 30) est un capteur capacitif (26) ou un capteur optique (31).

18. Dispositif selon l'une des revendications précédentes 7, 8 ou 10 à 17, caractérisé en ce que le dispositif de détection d'oscillations (18 ; 30, 31) est disposé après un guide-fil (7) qui est disposé en aval du dispositif de torsion (1) par rapport à la direction de circulation du fil.
